Europäisches Patentamt

European Patent Office

Office européen des brevets

(19)

(11) Veröffentlichungsnummer: **0 022 946**

**A1**

(12) **EUROPÄISCHE PATENTANMELDUNG**

(21) Anmeldenummer: **80103656.7**

(22) Anmeldetag: **27.06.80**

(51) Int. Cl.³: **G 01 N 7/00**
**G 01 N 33/44**

(30) Priorität: **18.07.79 DE 2929013**

(43) Veröffentlichungstag der Anmeldung:
**28.01.81 Patentblatt 81/4**

(84) Benannte Vertragsstaaten:
**BE FR GB IT NL**

(71) Anmelder: **Krauss-Maffei Aktiengesellschaft**
**Krauss-Maffei-Strasse 2**
**D-8000 München 50(DE)**

(72) Erfinder: **Hölzl, Emil, Ing.(grad.)**
**Klessingweg 4**
**D-8000 München 50(DE)**

(54) **Verfahren und Vorrichtung zum Messen der Gasbeladung von Flüssigkeiten.**

(57) Bei einem Verfahren zur Messung der Gasbeladung einer Flüssigkeit, insbesondere einer gasbeladenen flüssigen Kunststoffkomponente für die Herstellung von Schaumkunststoff wird zwecks Schaffung eines kontinuierlich arbeitenden Meßverfahrens hoher Genauigkeit, das auch bei unter hohem Druck stehender Flüssigkeit verwendet werden kann, die Flüssigkeit Druckschwingungen ausgesetzt, deren Amplituden $\Delta p$ und/oder deren Phasenlage die Meßgröße bilden.

Fig. 1

EP 0 022 946 A1

Croydon Printing Company Ltd.

- 1 -

Krauss-Maffei
Aktiengesellschaft
8000 München 50                TK 221d


Verfahren und Vorrichtung zum Messen
der Gasbeladung von Flüssigkeiten


Die Erfindung bezieht sich auf ein Verfahren und eine Vorrichtung zum Messen der Gasbeladung einer Flüssigkeit, insbesondere einer gasbeladenen flüssigen Kunststoffkomponente für die Herstellung von Schaumkunststoff.

Bei derartigen Herstellungverfahren, wie z.B. bei der Verarbeitung des Zweiphasensystems Polyol-Luft zu Polyurethanschäumen, wird die Schaumbildungsphase durch die eingeschlossenen Luft- bzw. Gasbläschen positiv beeinflußt. Die Qualität des Endproduktes hängt dabei unmittelbar vom Grad der Luftbeladung und dessen Konstanz ab. Um bestimmte Qualitätseigenschaften des Schaumkunststoffs zuverlässig einhalten zu können, wäre es daher erforderlich, den Gasbeladungsgrad zum einen exakt ermitteln zu können und zum anderen in engen Grenzen konstant halten zu können.

- 2 -

Dies ist mit den bisher angewandten
Methoden und Vorrichtungen lediglich in
sehr unzureichendem Maße möglich, da
die Messung nur an einer bestimmten
Flüssigkeitsmenge durchgeführt wird, die
aus dem rücklaufseitigen Leitungsbereich
einer Kunststoff verarbeitenden Maschine
abgezweigt wird. Die entnommene Flüssigkeitsprobe steht dabei unter dem im
rücklaufseitigen Leitungsbereich herrschenden Systemdruck, der wesentlich
niedriger ist, als der Druck im zulaufseitigen bzw. hochdruckseitigen Leitungsbereich. Das mittels eines, eine Membraneinrichtung aufweisenden Meßgerätes erhaltene Meßergebnis muß somit noch über Näherungswerte, die das spezifische Gas-Lösungsvermögen der flüssigen Kunststoffkomponente
im hochdruckseitigen Leitungsbereich beschreiben, korrigiert werden.

Abgesehen davon, daß dieses Meßverfahren
verhältnismäßig ungenau ist, läßt sich
damit wegen der diskontinuierlichen Arbeitsweise auch nicht die für gleichbleibende Qualitätseigenschaften erforderliche
Unveränderlichkeit des Gasbeladungsgrades
erzielen.

- 3 -

Der Erfindung liegt die Aufgabe zugrunde, ein kontinuierlich arbeitendes, hochdruckseitig einsetzbares Meßverfahren hoher Genauigkeit zu schaffen.

Diese Aufgabe wird dadurch gelöst, daß die Flüssigkeit Druckschwingungen ausgesetzt wird, deren Amplituden und/oder die Phasenlage die Meßgröße bilden.

Die Erfindung eignet sich insbesondere für den Einsatz eines automatischen Regelkreises, mit Hilfe dessen der Schwankungsbereich im Grad der Gasbeladung in engen Grenzen gehalten werden kann.

Weitere Merkmale und Vorteile der Erfindung ergeben sich aus der nachfolgenden Beschreibung eines Ausführungsbeispiels anhand der Zeichnung in Verbindung mit den Ansprüchen.

Es zeigen:
Fig. 1 ein Funktionsschema einer erfindungsgemäß ausgestatteten Maschine zur Herstellung von Schaumkunststoff und
Fig. 2 ein Diagramm des Verlaufs der Amplitudenwerte über der Dichte bzw. dem Volumenprozentanteil des in der flüssigen Komponente enthaltenen Gases.

In dem Funktionsschema ist mit 1 eine Verdrängerpumpe bezeichnet, die auf der Saugseite über eine Rohrleitung 2 mit einem Tank 3 verbunden ist. Dieser enthält eine reine oder mit Gas beladene, flüssige Kunststoffkomponente, beispielsweise Polyol. Auf der Druckseite der Verdrängerpumpe führt eine Hochdruckrohrleitung 4 zu einer Mischeinheit 5, die über eine Rückführleitung 6 mit dem Tank 3 verbunden ist. Die Hochdruckleitung 4 und die Rückführleitung 6 sind über eine weitere Leitung 7 verbunden, in der sich ein Luftbeladungsblock 8 befindet, dessen Funktion über ein Stellglied 9 steuerbar ist.

An der Hochdruckleitung 4 ist ein Druckmeßfühler 10, beispielsweise ein piezoelektrisches Element oder ein Dehnmeßstreifen angeordnet. In einer nachgeschalteten Meßwertaufbereitung 11 wird die Amplitude $\Delta p$ der als Pulsationsdruckschwankung in Erscheinung tretenden Druckschwankung herausgefiltert. Dieser Meßwert kann ggf. in einem Verstärker 12 noch verstärkt werden, bevor er in einen Meßumformer 13 eingegeben wird. Dieser enthält eine elektronische Speicherschaltung durch die der Meßwert aus der Druckschwingung entsprechend eingespeicherter Eichwerte derart gewandelt wird, daß das Ausgangssignal eine unmittelbare Aussage über den Grad der Gasbeladung der Flüssigkeit in Dichtewerten bzw. Volumenprozentanteilen ergibt.

- 5 -

Vom Meßumformer 13 zweigt eine Leitung 14 zu einer Anzeigevorrichtung 15 zur Kontrolle der Gasbeladungswerte ab. Die Ausgangssignale werden ebenfalls in eine Vergleicherschaltung 16 eingegeben in der ein gewünschter Sollwert S mit Grenzwerten einstellbar ist, so daß nach Vergleich mit dem vom Meßumformer 13 erhaltenen Istwert bzw. dessen Ausgangssignal über eine Steuerleitung 17 ein entsprechender Steuerimpuls zum Stellglied 9 ergeht. Dieses betätigt den Luftbeladungsblock 8 durch den über eine nicht dargestellte Gasdruckquelle Gas G in die flüssige Kunststoffkomponente eingebracht werden kann. Bei Anwendung der erfindungsgemäßen Maßnahmen und Vorrichtungen läßt sich somit ein schnell ansprechender und präzise wirkender Regelvorgang erzielen, wodurch der Gasbeladungsgrad der Flüssigkeit in vorgegebenen Grenzen konstant gehalten werden kann, was sich insbesondere bei der Herstellung von Schaumkunststoffteilen in einer Qualitätssteigerung auswirkt.

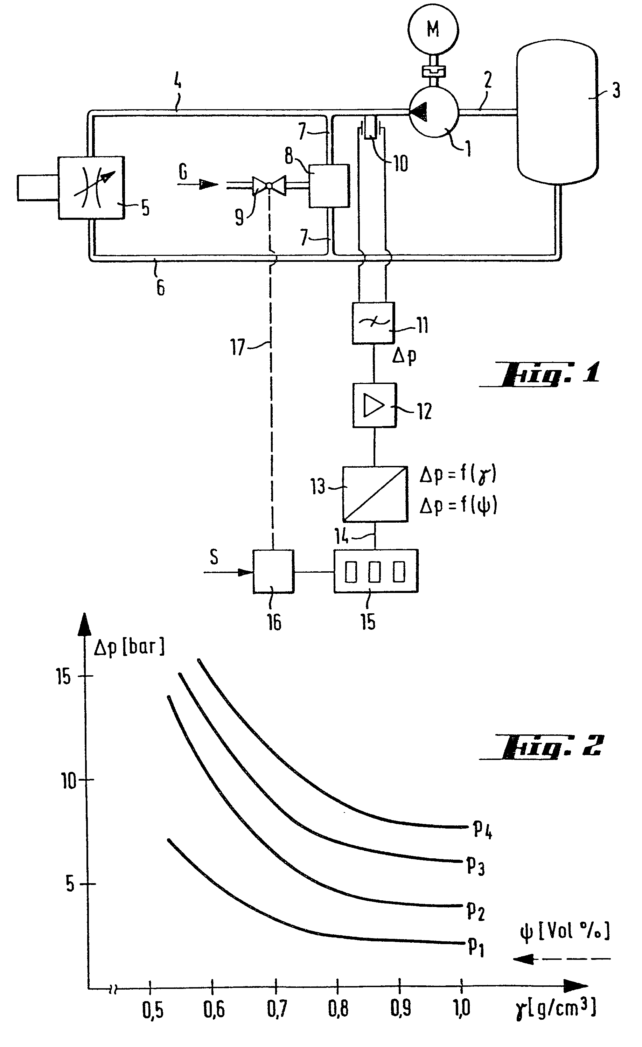

Das in Fig. 2 dargestellte Diagramm zeigt die gemäß der vorliegenden Erfindung genutzten Gesetzmäßigkeiten auf, nach denen der Meßwert in der Speicherschaltung des Meßumformers 13 gewandelt wird. Man geht dabei von der Erkenntnis aus, daß das System Flüssigkeit -Gas den Gesetzen des Zweimassenschwingers gehorcht, d.h., daß sich mit zunehmendem Gasanteil die Kompressibilität und die Masse der harmonisch erreg-

- 6 -

ten Flüssigkeitssäule ändern und die Amplitude $\Delta$ p̄ der Druckschwingung zunimmt.

In dem Diagramm sind in Funktion der Dichtewerte der gasbeladenen Flüssigkeit die Amplitudenwerte $\Delta$ p aufgetragen und zwar für verschiedene Arbeitsdrücke $p_1$, $p_2$, $p_3$ und $p_4$, denen sich dann jeweils die durch das Betriebsverhalten der Förderpumpe 1 bedingten Druckschwankungen mit der Amplitude $\Delta$ p überlagern. Das Diagramm gibt für Arbeitsdrücke von $p_1$ = 50 bar, $p_2$ = 100 bar, $p_3$ = 150 bar und $p_4$ = 200 bar praktisch gemessene Werte wieder, die somit Eichkurven darstellen, nach denen die Speicherschaltung des Meßumformers 13 zu programmieren ist. Die Eichkurven werden empirisch für beliebige Arbeitsdrücke erstellt. Aus den Dichtewerten lassen sich sodann in einfacher Weise die Volumenprozentanteile $\psi$ des in der Flüssigkeit gelösten Gases herleiten.

In Vorrichtungen, in denen keine Pulsationsdruckschwankungen erzeugende Förderpumpe vorhanden ist, kann eine gesonderte Anlage zur Erzeugung von Druckschwingungen vorgesehen werden.

- 7 -

Patentansprüche

1. Verfahren zum Messen der Gasbeladung einer
Flüssigkeit, insbesondere einer gasbeladenen, flüssigen Kunststoffkomponente für
die Herstellung von Schaumkunststoff,
d a d u r c h   g e k e n n z e i c h n e t ,
daß die Flüssigkeit Druckschwingungen ausgesetzt wird, deren Amplituden $\Delta$ p und/oder
deren Phasenlage die Meßgröße bilden.

2. Verfahren nach Anspruch 1, d a d u r c h
g e k e n n z e i c h n e t , daß die
Flüssigkeit einer harmonisch erregten
Druckschwingung ausgesetzt wird.

3. Verfahren nach den Ansprüchen 1 und 2,
d a d u r c h   g e k e n n z e i c h n e t ,
daß die Amplituden $\Delta$ p und/oder die Phasenlage einer von einer Förderpumpe (1) erzeugten Druckschwingung die Meßgröße bilden.

4. Verfahren nach den Ansprüchen 1 bis 3,
d a d u r c h   g e k e n n z e i c h n e t ,
daß der Meßwert der Meßgröße in einen
elektrischen Spannungs- oder Stromausschlag
umgeformt wird.

5. Verfahren nach Anspruch 4, d a d u r c h
g e k e n n z e i c h n e t , daß der
Meßwert in einem, eine elektronische Speicherschaltung aufweisenden Meßumformer (13)
entsprechend einer eingespeicherten Eichkurve gewandelt wird, die die Amplitude
$\Delta$ p in Funktion der Dichte $\gamma$ bzw. des
prozentualen Gasanteils $\psi$ der gasbeladenen Flüssigkeit darstellt.

6. Verfahren nach Anspruch 5, d a d u r c h
g e k e n n z e i c h n e t , daß der
Meßwert in einem Meßumformer (13) gewandelt wird, in dessen Speicherschaltung
mehrere, jeweils einem bestimmten Arbeitsdruck ($p_1$, $p_2$, $p_3$, $p_4$) entsprechende Eichkurven eingespeichert sind.

7. Verfahren nach den Ansprüchen 5 und 6,
d a d u r c h   g e k e n n z e i c h n e t ,
daß der die Dichte $\gamma$ bzw. den prozentualen
Gasanteil $\psi$ repräsentierende, gewandelte
Meßwert in einer nachgeschalteten Anzeigevorrichtung (15) dargestellt wird.

8. Vorrichtung zur Durchführung des Verfahrens
nach den Ansprüchen 1 bis 7, d a d u r c h
g e k e n n z e i c h n e t ,   daß eine
Vorrichtung zur Erzeugung einer harmonischen Druckschwingung in der gasbeladenen
Flüssigkeit vorgesehen ist und daß ein
Druck-Meßwertgeber (10) zur Erfassung der
Druckschwingung angeordnet ist.

9. Vorrichtung nach Anspruch 8, d a d u r c h
g e k e n n z e i c h n e t ,   daß die
Vorrichtung zur Erzeugung der harmonischen
Druckschwingung eine Förderpumpe (1) darstellt, an deren Leitungen, vorzugsweise an der Hochdruckleitung (4) der Druck-
Meßwertgeber (10) angeordnet ist.


10. Vorrichtung nach den Ansprüchen 8 und 9,
d a d u r c h   g e k e n n z e i c h n e t ,
daß der Druck-Meßwertgeber (10) ein, einen
elektrischen Spannungs- oder Stromausschlag erzeugendes piezoelektrisches Element oder ein Dehnmeßstreifen ist.


11. Vorrichtung nach Anspruch 10, d a d u r c h
g e k e n n z e i c h n e t ,  daß dem
Druck-Meßwertgeber (10) ein eine elektronische Speicherschaltung aufweisender
Meßumformer (13) nachgeschaltet ist, die
entsprechend einer Eichkurve programmiert
ist, die die Amplitude $\Delta$ p in Funktion der
Dichte $\gamma$ bzw. des prozentualen Gasanteils
$\psi$ der gasbeladenen Flüssigkeit darstellt.


12. Vorrichtung nach Anspruch 11, d a d u r c h
g e k e n n z e i c h n e t ,  daß in der
Speicherschaltung des Meßumformers (13)
mehrere Eichkurven einprogrammiert sind,
von denen jede den Amplitudenwert $\Delta$ p
und/oder die Phasenanlage in Funktion der
Dichte $\gamma$ bzw. des prozentualen Gasanteils
$\psi$ der gasbeladenen Flüssigkeit jeweils
für einen bestimmten Arbeitsdruck ($p_1$, $p_2$,
$p_3$, $p_4$) darstellt und daß eine Schaltvorrichtung zur Zuweisung des Meßwertes
an die dem jeweiligen Arbeitsdruck ($p_1$, $p_2$,
$p_3$, $p_4$,) zugeordnete Eichkurve vorgesehen
ist.

13. Vorrichtung nach den Ansprüchen 11 und 12, d a d u r c h  g e k e n n z e i c h n e t , daß dem Meßumformer (13) eine digitale oder analoge Anzeigevorrichtung (15) nachgeschaltet ist.

14. Vorrichtung nach den Ansprüchen 11 bis 13, d a d u r c h  g e k e n n z e i c h n e t , daß dem Meßumformer (13) eine Vergleicherschaltung (16) mit einstellbarem Dichte-Sollwert (S) nachgeschaltet ist.

15. Vorrichtung nach Anspruch 14, d a d u r c h  g e k e n n z e i c h n e t , daß vom Regler (16) eine Steuerleitung (17) zum Stellglied (9) eines Regelorgans führt, durch das über einen Gasbeladungsblock (8) Gas in die Flüssigkeit einbringbar ist.

Fig. 1

$\Delta p$

$\Delta p = f(\gamma)$
$\Delta p = f(\psi)$

Fig. 2

0022946

Europäisches
Patentamt

**EUROPÄISCHER RECHERCHENBERICHT**

Nummer der Anmeldung

EP 80 10 3656

| | **EINSCHLÄGIGE DOKUMENTE** | | | KLASSIFIKATION DER ANMELDUNG (Int. Cl.) |
|---|---|---|---|---|
| Kategorie | Kennzeichnung des Dokuments mit Angabe, soweit erforderlich, der maßgeblichen Teile | betrifft Anspruch | | |
| | US - A - 3 738 154 (R.E. HENRY)<br>* Spalte 2, Zeilen 34-43; Spalte 6, Zeilen 20-53; Figuren 1,2 *<br>-- | 1 | | G 01 N 7/00<br>33/44 |
| A | FR - A - 2 325 922 (MASCHINENFABRIK HENNECKE GmbH et BAYER A.G.)<br>* Seite 5, Zielen 1-11 *<br>-- | 1 | | |
| A | DE - A - 2 455 257 (KABEL UND ME-TALLWERKE GUTEHOFFNUNGSHUTTE)<br>* Seite 5, Zeilen 1-10; Figur 1 *<br>-- | 1 | | **RECHERCHIERTE SACHGEBIETE (Int. Cl.)**<br><br>G 01 N 7/00<br>33/44<br>11/08<br>B 29 D 27/02<br>G 05 D 21/02 |
| A | FR - A - 2 255 159 (DEMAG KUNST-STOFFTECHNIK)<br>* Seite 8, Zeilen 3-20 *<br>-- | 1 | | |
| A | FR - A - 2 390 721 (KLINGER A.G.)<br>* Seite 1, Zeile 5 - Seite 2, Zeile 9; Figur 1 *<br>---- | 1 | | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 16-10-1980 | ANTHONY |

EPA form 1503.1 06.78